# EUROPÄISCHE PATENTSCHRIFT

(11) **EP 1 513 500 B1**
(45) Veröffentlichungstag und Bekanntmachung des Hinweises auf die Patenterteilung: **21.01.2009**
(21) Anmeldenummer: 03755931.7
(22) Anmeldetag: 19.05.2003
(51) Int. Cl.: A61K 9/10, A61K 47/48, A61K 31/4709

(54) **PHARMAZEUTISCHE ZUBEREITUNGEN ZUR ORALEN ANWENDUNG ENTHALTEND PRADOFLOXACIN-BELADENE IONENTAUSCHERHARZE SOWIE STRUKTURVISKOSE GELBILDNER ALS VERDICKER**
PHARMACEUTICAL PREPARATIONS FOR ORAL ADMINISTRATION, CONTAINING ION-EXCHANGE RESINS LOADED WITH PRADOFLOXACIN AND INTRINSICALLY VISCOUS GELLING AGENTS AS THICKENING AGENTS
PREPARATIONS PHARMACEUTIQUES POUR ADMINISTRATION ORALE, CONTENANT DES RESINES ECHANGEUSES D'IONS CHARGEES DE PRADOFLOXACINE AINSI QUE DES GELIFIANTS A VISCOSITE INTRINSEQUE COMME EPAISSISSANTS

(30) Priorität: 31.05.2002 DE 10224086
(43) Veröffentlichungstag der Anmeldung: 16.03.2005
(73) Patentinhaber: Bayer Animal Health GmbH, 51368 Leverkusen (DE)
(72) Erfinder: MERTIN, Dirk, 40764 Langenfeld (DE); EDINGLOH, Markus, 51379 Leverkusen (DE); DAUBE, Gert, 51766 Engelskirchen (DE)
(86) Internationale Anmeldenummer: PCT/EP2003/005228
(87) Internationale Veröffentlichungsnummer: WO 2003/101422

(56) Entgegenhaltungen:
- EP-A- 0 295 495
- EP-A- 0 564 154
- EP-A- 0 911 039
- WO-A-01/05431
- WO-A-03/007995

## Beschreibung

Die vorliegende Erfindung betrifft pharmazeutische Zubereitungen gemäß Anspruch 1 zur oralen Anwendung, welche Pradofloxacin enthalten, das an einen Ionenaustauscher gebunden sind. Zur Verbesserung der physikalischen Stabilität und der Akzeptanz, insbesondere bei Tieren, ist ein strukturviskoser Gelbildner als Verdicker enthalten.

Es ist seit langem bekannt, pharmazeutische Wirkstoffe an Ionentauscherharze zu binden, um beispielsweise Wirkstoffe mit ausgeprägtem Eigengeruch besser anwendbar zu machen (CH 383552). Es ist auch bekannt, pharmazeutische Wirkstoffe an Ionentauscherharze zu binden, um eine gleichmäßige Freigabe des Wirkstoffes über eine längere Zeit zu beeinflussen (DE 3 028 082). Es ist ferner bekannt, anthelmintische Wirkstoffe an Ionentauscherharze zu binden, um den Geschmack der Wirkstoffe zu beeinflussen (DE 3 028 082). Durch die Bindung an Ionentauscherharze kann auch der bittere Geschmack von Chinolon-Antibiotika maskiert werden, so dass eine Applikation bei Tieren ermöglicht wird (EP-A-295 495).

In der vorgenannten Schrift werden Formulierungen von bitter schmeckenden Chinoloncarbonsäurederivaten, die an Ionentauscherharze gebunden werden, sowie deren Herstellung beschrieben. Als Ionentauscherharze kommen schwach saure kationische Typen in Frage, wobei deren Matrix gelförmig bzw. makroporös sein kann. Als Basismonomere für die Ionentauscher kommen polymerisierbare Monomere in Betracht, welche durch entsprechende Seitenketten zu Kationentauscherharzen funktionalisiert werden können. Die Ionentauscher sind unter den Handelsnamen Lewatit^{®}, Amberlite^{®}, Purolite^{®} oder Dowex^{®} bekannt. Entsprechende Formulierungen sind in der Veterinärmedizin als Fütterungsarzneimittel für Schweine beschrieben.

Die oft ungünstige Oberflächenstruktur von Ionentauschern führt in flüssigen Suspensionen häufig zu einer starken Verbackung des Bodensatzes nach Sedimentation der Partikel.

Unsere Patentanmeldung WO 03/007995 beschreibt die Stabilisierung derartiger Ionentauscher durch Mahlung, so dass mindestens 90 % der Partikel kleiner als 50 µm sind. Vermutlich durch Abrundung der unregelmäßigen Oberfläche lassen sich dann die gebildeten Sedimente leicht wieder aufschütteln. Derartige Mahlprozesse sind jedoch sehr aufwändig und verteuern die Herstellung entsprechender Zubereitungen deutlich.

Bekannt ist ferner, pharmazeutische Zubereitungen durch Einsatz von Gelbildnern (z.B. Hydroxyethylcellulose, Hydroxypropylcellulose, Hydroxymethylpropylcellulose) soweit zu verfestigen, dass keine Sedimentation mehr auftreten kann. Allerdings fließen derartige halbfeste Zubereitungen nicht mehr unter ihrer eigenen Gewichtskraft. Flüssige Arzneiformen lassen sich hiermit nur schwierig realisieren.

Es ist ferner bekannt, dass flüssige, wässrige Suspensionen von Ionentauscherharzen mit strukturviskosen Gelbildnern (z.B. Polyacrylsäure, Xanthan, Traganth, Na-carboxymethylcellulose, Bentonit) stabilisiert werden können. So beschreiben US 6 146 622 und US 2 002 035 154 mit Polyacrylsäure (Carbopol 974 P) oder Xanthan stabilisierte Suspensionen eines Kationentauschers in Wasser. US 5 612 026 beschreibt ein Mixgetränk, welches einen Anionentauscher und Xanthan enthält. Balkus et al. (Langmuir, 12, 6277-6281 (1996)) publizierten die Stabilisierung von Gadolinium-haltigen Hectorit-Suspensionen mit Xanthan. Weitere Beschreibungen wässriger Ionentauschersuspensionen, die mit strukturviskosen Polymeren gegen Sedimentation stabilisiert werden, finden sich in JP 05 279 245, JP 05 279 246, Sprockel et al., Drug Dev. Ind. Pharm. 15, 1275-1287 (1989), EP 139 881, JP 01 071 823, JP 01 071 822 sowie JP 63 230 636. Derartige Suspensionen besitzen im Ruhezustand eine hohe Viskosität, wodurch eine Sedimentation der suspendierten Ionentauscherpartikel verhindert wird. Unter Krafteinwirkung verflüssigt sich das System und kann somit leicht gepumpt oder gespritzt werden.

Überraschenderweise wurde nun gefunden, dass Suspensionen eines wirkstoffbeladenen Ionentauschers, die mit einem strukturviskosen Polymer verdickt wurden, ein besonders gutes Mundgefühl aufweisen und nach oraler Gabe sehr gut akzeptiert und vertragen werden. Solche Suspensionen sind weiterhin stabil gegen Sedimentation der festen Bestandteile und haben vorteilhafte Fließeigenschaften, die bei einem Mittel zur oralen Gabe wünschenswert sind. Nach Aufnahme in den Magen wird der pharmazeutische Wirkstoff rasch vom Ionentauscher abgelöst. Das pharmakokinetische Profil ist daher gegenüber ungebundenem Wirkstoff nur unwesentlich verändert.

Die Erfindung betrifft daher
• Flüssige pharmazeutische Zubereitung enthaltend Pradofloxacin gebunden an einen Ionentauscher, wobei der beladene Ionentauscher in einem Wasser enthaltenden Trägermedium dispergiert ist, das Trägermedium einen oder mehrere strukturviskose Gelbildner enthält, mit der maßgabe, dass der struktur viskose Gelbildner nicht Hydroxyethylcellulose ist.
• Die Verwendung der vorstehend genannten pharmazeutischen Zubereitung zur Herstellung von Arzneimitteln zur oralen Applikation.

Als strukturviskose Gelbildner können beispielsweise Mikrokristalline Cellulose, Celluloseether (Methylcellulose, Hydroxyethylcellulose, Hydroxypropylcellulose, Methylhydroxypropylcellulose, Na-carboxymethylcellulose), Xanthan, Traganth, Guarmehl, Gummi arabicum, Stärke und Stärkederivate wie Na-carboxylmethylstärke, Gelatine, hochdisperses Siliziumdioxid (z.B. Aerosil), Polyacrylsäure, Aluminiumstearat oder Bentonit verwendet werden. Es ist auch möglich, die genannten Gelbildner miteinander zu kombinieren. Hochdisperses Siliziumdioxid findet bevorzugt Verwendung bei der Verdickung nicht-wässriger Trägerflüssigkeiten. Für die Verdickung wässriger Trägerflüssigkeiten werden bevorzugt mikrokristalline Cellulose, Celluloseether, Xanthan, Polyacrylsäure und Bentonit oder Mischungen der genannten Gelbildner verwendet. Besonders bevorzugt ist dabei die Verwendung von mikrokristalliner Cellulose, Carboxymethylcellulose-Na, Xanthan, Polyacrylsäure und Bentonit.

Die Ionenaustauscherharze können beispielsweise eine gelförmige oder, makroporöse Matrix aufweisen. Als Basismonomere für die Ionenaustauscher kommen polymerisierbare Monomere in Betracht, welche durch entsprechende Funktionalisierung zu Ionenaustauscherharzen umgewandelt werden können. Als Monomere seien beispielsweise (Meth)-acrylsäureester, (Meth)-acrylnitril sowie Styrol-Abkömmlinge genannt. Als weitere Comonomere werden zur Herstellung der Basispolymere Polyvinylverbindungen wie beispielsweise Divinylbenzol, Ethylenglykoldimethacrylat oder Methylenbisacrylamid eingesetzt. Auch Kondensationsharze, die zu Ionenaustauschern führen, sind geeignet, beispielsweise Phenol-Formaldehyd-Harze mit entsprechenden funktionellen Gruppen.

Die verwendbaren Ionenaustauscher sind bekannt. Nähere Angaben zu verschiedenen Ionenaustauschertypen und ihrer Herstellung finden sich beispielsweise in Ullmann's Encyclopedia of Industrial Chemistry (Release 2001, 6th Edition). Die bevorzugten makroporösen Harze können unterschiedliche Porenvolumen aufweisen. Der Vernetzungsgrad der geeigneten Ionenaustauscherharze sollte bevorzugt bis zu 20 % und besonders bevorzugt bis zu 12 % betragen. Die Kunstharze liegen üblicherweise in Korngrößen von 1 bis 300 µm, bevorzugt von 10 bis 200 µm vor. Handelsübliche Ionenaustauscherharze sind z. B. Lewatit^{®}, Amberlite^{®}, Dowex^{®} und Purolite^{®}.

Zur Bindung basischer bzw. kationischer Wirkstoffe können saure Ionentauscher verwendet werden.

Als stark saure Ionenaustauscher werden bevorzugt solche auf Poly(styrol, divinylbenzol)sulfonsäure-Basis eingesetzt. Als Beispiele seien genannt:
• Amberlite IRP 69: Poly(styrol, divinylbenzol) sulfonsäure in der Na-Form, Übliche Partikelgröße: 10-25% > 75 µm, max. 1% > 150 µm, K-AustauschKapazität: 110 -135 mg/g entsprechend 2,75 - 3,38 eq/kg
• Purolite C 100 H MR: Poly(styrol, divinylbenzol) sulfonsäure in der H-Form, Übliche Partikelgröße: max. 1 % > 150 µm, Austauschkapazität: mindestens 3,2 eq/kg
• Purolite C 100 MR: Poly(styrol, divinylbenzol) sulfonsäure in der Na-Form, entspricht Amberlite IRP 69
• Lewatit Catalyst K 1481: Poly(styrol, divinylbenzol) sulfonsäure in der H-Form, Übliche Partikelgröße: min. 97 % < 30 µm, Austauschkapazität.: 5,0 eq/kg
• Lewasorb SW 12: Poly(styrol, divinylbenzol) sulfonsäure in der Na-Form, entspricht ansonsten Lewatit K 1481.

Als schwach saure Kationentauscher werden insbesondere solche auf Methacrylsäure-Divinylbenzol-Copolymer-Basis eingesetzt. Beispiele sind:
• Amberlite IRP 64: : Methacrylsäure-Divinylbenzol-Copolymer in der H-Form, Übliche Partikelgröße: 15-30 % > 75 µm, max. 1 % > 150 µm, Austauschkapazität: min. 10 eq/kg
• Purolite C 115 K MR: Methacrylsäure-Divinylbenzol-Copolymer in der Kalium-Form, übliche Partikelgröße max. 1 % > 150 µm
• Purolite C 115 H MR: Methacrylsäure-Divinylbenzol-Copolymer in der H-Form; ansonsten wie Purolite C 115 K MR.
• Lewatit CNP 105: makroporöses Methacrylsäure-Divinylbenzol-Copolymer in der H-Form, Austauschkapazität min. 1,4 eq/l.

Zur Bindung saurer bzw. anionischer Wirkstoffe können Anionentauscher verwendet werden.

Als Anionenaustauscher werden bevorzugt Polystyrolharze mit Amin- bzw. Ammonium-Seitengruppen eingesetzt. Als Beispiele seien genannt:
• Purolite A 430 MR: Poly(styrol-divinylbenzol)-trimethylammoniumchlorid, Austauschkapazität 3,7 - 4,8 eq/kg
• Lewatit MP 500: Poly(styrol-divinylbenzol)-trimethylammoniumchlorid, Austauschkapazität min. 1,1 eq/l
• Lewatit MP 62 WS: Poly(styrol-divinylbenzol)-dimethylamin, Austauschkapazität min. 1,7 eq/l
• Duolite AP143/1093: Poly(styrol-divinylbenzol)-trimethylammoniumchlorid, Austauschkapazität 3,7 - 4,8 eq/kg

Die erfindungsgemäße Formulierung enthält die in WO 97/31001 beschriebene Verbindung 8-Cyan-1-cyclopropyl-7-((1S,6S)-2,8-diazabicyclo[4.3.0]nonan-8-yl)-6-fluor-1,4-dihydro-4-oxo-3-chinolincarbonsäure (Pradofloxacin) mit der Formel

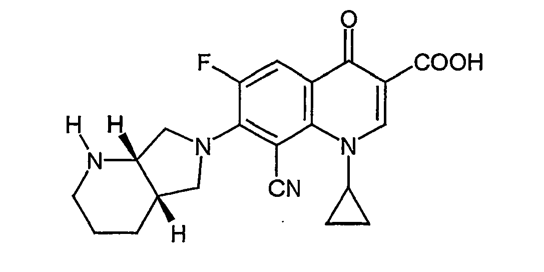

Die Beladung der Ionentauscherharze mit Wirkstoff erfolgt in Wasser oder polaren organischen Lösungsmitteln, wie z.B. Alkoholen, wie Propylenglykol oder Ethanol, Glycerol, Ketonen, wie Aceton oder Gemische derselben. Besonders bevorzugt sind Wasser sowie Alkohol-Wasser-Mischungen. Ionentauscher und Wirkstoff werden dabei solange in dem Medium bei Raumtemperatur oder erhöhter Temperatur gerührt, bis der Wirkstoff vollständig gebunden ist. Die Beladung des Ionentauschers mit Wirkstoff und die Formulierung der Arzneiform kann auch in einem Schritt erfolgen.

Sofern der wirkstoffbeladene Ionentauscher in einem mit Wasser bzw. polaren Lösungsmittel nicht mischbaren, lipophilen Trägermedium dispergiert werden soll, so muss er zunächst vom wässrigen Beladungsmedium getrennt und getrocknet werden. Anschließend kann der beladene Ionetauscher in das Trägermedium eingearbeitet werden. Als lipophiles Trägermedium können beispielsweise fette Öle, Paraffin- oder Silikonöle verwendet werden. Hierbei ist die Verwendung von fetten Ölen, beispielsweise mittelkettige Triglyceride, Sesamöl, Erdnussöl oder Sojaöl bevorzugt.

Die erfindungsgemäßen pharmazeutischen Zubereitungen eignen sich generell für die Anwendung bei Mensch und Tier. Bevorzugt werden sie in der Tierhaltung und Tierzucht bei Nutz-, Zucht-, Zoo-, Labor-, Versuchs- und Hobbytieren eingesetzt.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär sowie Vögel wie z.B. Hühner, Gänse, Puten, Enten, Tauben und Vogelarten für Heim- und Zoohaltung.

Zu Labor- und Versuchstieren gehören Mäuse, Ratten, Meerschweinchen, Goldhamster, Hunde und Katzen.

Zu den Hobbytieren gehören Kaninchen, Hamster, Meerschweinchen, Mäuse, Pferde, Reptilien, entsprechende Vogelarten, Hunde und Katzen.

Weiterhin seien Fische genannt, und zwar Nutz-, Zucht-, Aquarien- und Zierfische aller Altersstufen, die in Süß- und Salzwasser leben. Zu den Nutz- und Zuchtfischen zählen z.B. Karpfen, Aal, Forelle, Weißfisch, Lachs, Brachse, Rotauge, Rotfeder, Döbel, Seezunge, Scholle, Heilbutt, Japanese yellowtail (Seriola quinqueradiata), Japanaal (Anguilla japonica), Red seabream (Pagurus major), Seabass (Dicentrarchus labrax), Grey mullet (Mugilus cephalus), Pompano, Gilthread seabream (Sparus auratus), Tilapia spp., Chichliden-Arten wie z.B. Plagioscion, Channel catfish. Geeignet sind die erfindungsgemäßen Mittel zur Behandlung von Fischbrut, z.B. Karpfen von 2 bis 4 cm Körperlänge, sowie in der Aalmast.

Bevorzugt werden die erfindungsgemäßen Zubereitungen bei Hobbytieren wie Hamster, Kaninchen, Meerschweinchen, Katzen und Hunden eingesetzt. Insbesondere eignen sie sich für die Anwendung bei Katzen.

Die Anwendung kann sowohl prophylaktisch als auch therapeutisch erfolgen.

Die erfindungsgemäßen Zubereitungen werden bevorzugt oral verabreicht.

Für Tiere geeignete Arzneimittelzubereitungen sind z.B. solche, bei denen die Geschmacksverbesserung bei der Aufnahme eine Rolle spielt oder bei denen eine verzögerte Wirkstoff-Freisetzung nach der Applikation angestrebt wird.

Zur Herstellung von Suspensionen werden die wirkstoffbeladenen Harze in einem flüssigen Trägermedium möglichst homogen verteilt, gegebenenfalls unter Zuhilfenahme von anderen Hilfsstoffen wie Netzmittel. Mit Hilfe strukturviskoser Gelbildner werden die Suspensionen angedickt. Weitere Hilfsstoffe wie Netzmittel, Antioxidantien, Konservierungsmittel, Farbstoffe sowie Geschmackstoffe oder Aromen können ebenfalls enthalten sein.

Die erfindungsgemäßen Zubereitungen enthalten den wirkstoffbeladenen Ionenaustauscher üblicherweise in einer Menge von 1 bis 50 Gew.-%, bevorzugt 5 bis 30 Gew.-%, besonders bevorzugt 5 bis 25 Gew.-%, bezogen auf das Gesamtgewicht der Zubereitung.

Als Trägerflüssigkeiten seien genannt Wasser, Mischungen aus Wasser und wassermischbaren, organischen Lösungsmitteln (z.B. Alkohole wie Ethanol, Isopropanol, Propylenglykol, Glycerol, Polyethylenglykol) sowie lipophile Trägerflüssigkeiten (z.B. fette Öle, Paraffin- oder Silikonöle). In den erfindungsgemäßen Zubereitungen ist die Trägerflüssigkeit in einer für die gewünschte Konsistenz geeigneten Menge enthalten, üblicherweise 10 bis 98 Gew.-%, bevorzugt 20 bis 90 Gew.-% bezogen auf das Gesamtgewicht der Zubereitung.

Als Netzmittel (Dispergiermittel) seien genannt:
• anionaktive Tenside einschließlich Emulgatoren wie Na-Laurylsulfat, Fettalkoholethersulfate, Mono/Dialkylpolyglykoletherorthophosphorsäureester-Monoethanolaminsalz, Ligninsulfonate oder Dioctylsulfosuccinat
• kationaktive Tenside, einschließlich Emulgatoren wie Cetyltrimethylammoniumchlorid
• ampholytische Tenside, einschließlich Emulgatoren wie Di-Na-N-lauryl-βiminodipropionat oder Lecithin
• nicht ionogene Tenside, einschließlich Emulgatoren, wie polyoxyethyliertes Rizinusöl, polyoxyethylierte Sorbitan-Fettsäureester, Sorbitan-Fettsäureester, Glycerinmono- und -diglyceride, Polyoxyethylen-Fettsäureester, Polyoxyethylen-Fettalkoholeteher, Alkylphenolpolyglykolether, Polyethylen-Polypropylen-Blockcopolymere.

Das Netzmittel ist in den erfindungsgemäßen Zubereitungen üblicherweise in einer Menge von 0,01 bis 10 Gew.-%, bevorzugt 0,1 bis 2 Gew.-% bezogen auf das Gesamtgewicht der Zubereitung enthalten.

Weitere Hilfsstoffe sind z.B.:
• Konservierungsmittel, wie z.B. p-Hydroxybenzoesäureester, Sorbinsäure, Benzoesäure, Propionsäure, Ameisensäure oder deren Salze. Das Konservierungsmittel ist in den erfindungsgemäßen Zubereitungen üblicherweise in einer Menge von 0,05 bis 2 Gew.-%, bevorzugt 0,1 bis 1 Gew.-% bezogen auf das Gesamtgewicht der Zubereitung enthalten.
• Farbstoffe, d.h. alle zur Anwendung am Menschen oder Tier zugelassenen Farbstoffe, die gelöst oder suspendiert sein können. Farbstoffe sind in den erfindungsgemäßen Zubereitungen üblicherweise in einer Menge von 0,001 bis 5 Gew.-%, bevorzugt 0,01 bis 2 Gew.-% bezogen auf das Gesamtgewicht der Zubereitung enthalten.
• Antioxidantien wie z.B. Sulfite oder Metabisulfite wie Kaliummetabisulfit, Ascorbinsäure, Butylhydroxytoluol, Butylhydroxyanisol, Tocopherole. Antioxidantien sind in den erfindungsgemäßen Zubereitungen üblicherweise in einer Menge von 0,001 bis 5 Gew.-%, bevorzugt 0,01 bis 2 Gew.-% bezogen auf das Gesamtgewicht der Zubereitung enthalten.
• Geschmacksstoffe oder Aromen sind die üblicherweise bei Arzneimitteln eingesetzten, beispielsweise Vanillin. Geschmacksstoffe oder Aromen sind in den erfindungsgemäßen Zubereitungen üblicherweise in einer Menge von 0,001 bis 5 Gew.-%, bevorzugt 0,01 bis 2 Gew.-% bezogen auf das Gesamtgewicht der Zubereitung enthalten.

Bevorzugt handelt es sich bei den erfindungsgemäßen Zubereitungen um flüssige Suspensionen. Die Fließgrenze liegt zwischen 0 und 100 Pa, vorzugsweise zwischen 5 und 50 Pa. Die Viskosität bei 300 s⁻¹ beträgt zwischen 10 und 1000 mPa*s, vorzugsweise zwischen 50 und 500 mPa*s.

Die Verabreichung der erfindungsgemäßen Zubereitung kann separat oder zusammen mit dem Futter erfolgen.

Zum Futter zählen Einzelfuttermittel pflanzlicher Herkunft wie Heu, Rüben, Getreide, Getreidenebenprodukte, Einzelfuttermittel tierischer Herkunft wie Fleisch, Fette, Milchprodukte, Knochenmehl, Fischprodukte, ferner die Einzelfuttermittel wie Vitamine, Proteine, Aminosäuren, z.B. DL-Methionin, Salze wie Kalk und Kochsalz. Zum Futter zählen auch Ergänzungs-, Fertig- und Mischfuttermittel. Diese enthalten Einzelfuttermittel in einer Zusammensetzung die eine ausgewogene Ernährung hinsichtlich der Energie- und Proteinversorgung sowie der Versorgung mit Vitaminen, Mineralsalzen und Spurenelementen sicherstellen.

### Herstellungsbeispiele

### Beispiel 1

0,18 kg Methyl-p-hydroxybenzoat und 0,02 kg Propy-p-hydroxybenzoat werden in 75,0 kg heißem Wasser gelöst. Darin werden 0,3 kg Xanthan (Xantural 180, CP Kelco) und 0,3 kg Bentonit (Veegum, Vanderbildt) unter starkem Rühren eingearbeitet und eine Stunde bei 70°C gerührt. Nach Erkalten werden in dem entstandenen Sol 6,0 kg Pradofloxacin, 18,0 kg eines schwach sauren Ionentauschers (Amberlite IRP 64) und 1,0 kg Vanillin dispergiert. Der gesamte Ansatz wird anschließend mit einem Rotor-Stator homogenisiert. Es resultiert eine dickflüssige Suspension.

### Beispiel 2

Wie Beispiel 1, mit dem Unterschied dass 0,2 kg Sahne-Karamel-Aroma statt 1,0 kg Vanillin verwendet werden.

### Beispiel 3

0,2 kg Sorbinsäure und 0,02 kg Ascorbinsäure werden in 74,98 kg heißem Wasser gelöst. Darin werden 0,3 kg Xanthan (Xantural 180, CP Kelco) und 0,3 kg Bentonit (Veegum, Vanderbildt) unter starkem Rühren eingearbeitet und eine Stunde bei 70°C gerührt. Nach Erkalten werden in dem entstandenen Sol 6,0 kg Pradofloxacin, 18,0 kg eines schwach sauren Ionentauschers (Amberlite IRP 64) und 0,2 kg eines Sahne-Karamel-Aromas dispergiert. Der gesamte Ansatz wird anschließend mit einem Rotor-Stator homogenisiert. Es resultiert eine dickflüssige Suspension.

### Beispiel 4

Wie Beispiel 1, mit dem Unterschied dass 0,1 kg Vanille-Aroma statt 1,0 kg Vanillin verwendet werden.

### Beispiel 5

0,2 kg Sorbinsäure werden in 30,0 kg Propylenglykol gelöst. In diese Lösung wird 0,7 kg Xanthan (Xantural 180, CP Kelco) unter starkem Rühren eingearbeitet. In einem zweiten Behälter werden 2,5 kg Pradofloxacin, 0,02 kg Ascorbinsäure sowie 0,2 kg eines Vanillearomas in 61,58 kg Wasser gelöst. In der Lösung werden 10,0 kg schwach saurer Kationentauscher (Amberlite IRP 64) dispergiert. Unter Verwendung eines hochtourigen Rührers wird in diese Suspension die Xanthan/Propylenglykol-Dispersion eingearbeitet. Der gesamte Ansatz wird anschließend mit einem Rotor-Stator homogenisiert. Es resultiert eine dickflüssige Suspension.

### Beispiel 6

0,2 kg Sorbinsäure werden in 30,0 kg Glycerol gelöst. In diese Lösung wird 0,5 kg Xanthan (Xantural 180, CP Kelco) unter starkem Rühren eingearbeitet. In einem zweiten Behälter werden 6,0 kg Pradofloxacin in 48,3 kg Wasser gelöst. In der Lösung werden 10,0 kg schwach saurer Kationentauscher (Amberlite IRP 64) sowie 5,0 kg eines Fleischaromas dispergiert. Unter Verwendung eines hochtourigen Rührers wird in diese Suspension die Xanthan/Glycerol-Dispersion eingearbeitet. Der gesamte Ansatz wird anschließend mit einem Rotor-Stator homogenisiert. Es resultiert eine dickflüssige Suspension.

### Beispiel 7 (Vergleichsbeispiel)

5,00 kg Enrofloxacin und 20,00 kg Purolite C 100 H MR werden in 80,00 kg gereinigtem Wasser suspendiert und über mindestens 8 Stunden bei Raumtemperatur gerührt. Nach Sedimentation wird der Überstand abgelassen. Der Rückstand wird mit einem Filtertrockner bei 75°C getrocknet. Dazu parallel werden 0,384 kg Sorbinsäure in 146,496 kg heißem Wasser gelöst. In einem zweiten Behälter werden 0,96 kg Polyacrylsäure (Carbopol 974P, BFGoodrich) in 19,20 kg Glycerol dispergiert. 24,00 kg des getrockneten, beladenen Ionentauschers werden darin suspendiert.

### Beispiel 8

5,00 kg Pradofloxacin und 20,00 kg Purolite C 115 HMR werden in 75,00 kg gereinigtem Wasser suspendiert. Anschließend werden 0,25 kg Benzoesäure und 0,5 kg Bentonit (Veegum, Vanderbildt) unter starkem Rühren eingearbeitet und 1 Stunde auf 70°C erhitzt, so dass eine flüssige Suspension entsteht.

### Beispiel 9

0,2 kg Sorbinsäure werden in 30,0 kg Propylenglykol gelöst. In diese Lösung werden 2,25 kg einer Mischung aus Mikrokristalliner Cellulose und Na-carboxymethylcellulose (Avicel CL 611, FMC) unter starkem Rühren eingearbeitet. In einem zweiten Behälter werden 6,0 kg Pradofloxacin, 0,02 kg Ascorbinsäure sowie 0,2 kg eines Vanillearomas in 54,35 kg Wasser gelöst. In der Lösung werden 18,0 kg schwach saurer Kationentauscher (Amberlite IRP 64) dispergiert. Unter Verwendung eines hochtourigen Rührers wird in diese Suspension die Gelbildner/Propylenglykol-Dispersion eingearbeitet. Der gesamte Ansatz wird anschließend mit einem Rotor-Stator homogenisiert. Es resultiert eine dickflüssige Suspension.

### Beispiel 10

6,0 kg Pradofloxacin werden zusammen mit 18,0 kg Amberlite IRP 64 über 16 h in 72 kg Wasser gerührt. Nach Absetzen der suspendierten Partikel wird der Überstand abgegossen und der Rückstand bei 70°C getrocknet. Dieser wirkstoffbeladene Ionentauscher wird zusammen mit 1,0 kg Vanillin in 72,0 kg Mittelkettigen Triglyceriden (Miglyol 812) dispergiert. Nach Zugabe von 3,0 kg hochdispersem Siliziumdioxid (Aerosil 200) wird der Ansatz mit einem Rotor-Stator homogenisiert. Es resultiert eine dickflüssige Suspension.

### Biologisches Beispiel

### Palatabilitäts-Test

Die erfindungsgemäßen pharmazeutischen Zubereitungen zeichnen sich aufgrund ihres guten Mundgefühls, welches auch auf die besondere Konsistenz zurückzuführen ist, durch eine ausgezeichnete Palatabilität, z. B. bei Katzen, aus. Die folgende Tabelle 1 fasst die Ergebnisse eines entsprechenden Tests an Katzen zusammen:

**Tabelle 1: Palatabilität erfindungsgemäßer Beispiele 1- 4, 10 bei Katzen im Vergleich zu einer gut palatablen Referenzformulierung (nutri-plus Cat, virbac Tierarzneimittel, Bad Oldesloe), n = 36, Score: 1 = unakzeptabel, 2 = schwierige Applikation, 3 = mäßige Abwehr, 4 = leicht zu applizieren, 5 = sehr leicht zu applizieren Zusammensetzung der Beispiele: s.o.**

| **Beispiel** | **1** | **2** | **3** | **4** | **10** | **Referenz** |
|---|---|---|---|---|---|---|
| Palatabilitäts-score | 3,5 | 3,4 | 3,5 | 3,4 | 3,3 | 3,5 |
| Speicheln nach Applikation | 0% | 0% | 0% | 5,5% | 11,1 % | 0% |

## Patentansprüche

1. Flüssige pharmazeutische Zubereitung enthaltend Pradofloxacin gebunden an einen Ionentauscher, wobei der beladene Ionentauscher in einem Wasser enthaltenden Trägermedium dispergiert ist und das Trägermedium einen oder mehrere strukturviskose Gelbildner enthält, mit der Maßgabe, dass der strukturviskose Gelbildner nicht Hydroxyethylcellulose ist.

2. Pharmazeutische Zubereitung gemäß Anspruch 1, **dadurch gekennzeichnet, dass** sie als strukturviskosen Gelbildner mikrokristalline Cellulose, Xanthan, Polyacrylsäure, Bentonit oder einen Celluloseether ausgewählt aus Methylcellulose, Hydroxypropylcellulose, Methylhydroxypropylcellulose, Na-carboxymethylcellulose oder eine Mischung der genannten Gelbildner enthält.

3. Pharmazeutische Zubereitung gemäß Anspruch 2, **dadurch gekennzeichnet, dass** sie als strukturviskosen Gelbildner mikrokristalline Cellulose, Na-Carboxymethylcellulose, Xanthan, Polyacrylsäure oder Bentonit enthält.

4. Pharmazeutische Zubereitung gemäß Anspruch 2, **dadurch gekennzeichnet, dass** der strukturviskose Gelbildner Xanthan ist.

5. Verwendung pharmazeutischer Zubereitungen gemäß einem der vorstehenden Ansprüche zur Herstellung von Arzneimitteln zur oralen Applikation.

## Claims

1. Liquid pharmaceutical preparation comprising pradofloxacin bound to an ion exchanger, the loaded ion exchanger being dispersed in a water-comprising carrier medium and the carrier medium comprising one or more pseudoplastic gel formers, with the proviso that the pseudoplastic geel former is not hydroxyethylcellulose.

2. Pharmaceutical preparation according to Claim 1, **characterized in that** as pseudoplastic gel former it comprises microcrystalline cellulose, xanthan, polyacrylic acid, bentonite or a cellulose ether selected from methylcellulose, hydroxypropylcellulose, methylhydroxypropylcellulose. Na-carboxymethylcellulose or a mixture of the above gel formers.

3. Pharmaceutical preparation according to Claim 2, **characterized in that** as pseudoplastic gel former it comprises microcrystalline cellulose, Na-carboxymethylcellulose, xanthan, polyacrylic acid or bentonite.

4. Pharmaceutical preparation according to Claim 2, **characterized in that** the pseudoplastic gel former is xanthan.

5. Use of pharmaceutical preparations according to one of the preceding claims for producing medicaments for oral administration.

## Revendications

1. Préparation pharmaceutique liquide contenant de la pradofloxacine liée à un échangeur d'ions, l'échangeur d'ions chargé étant dispersé dans un milieu de support contenant de l'eau et le milieu de support contenant un ou plusieurs gélifiants pseudoplastiques, sous réserve que le gélifiant pseudoplastique ne soit pas de l'hydroxyéthylcellulose.

2. Préparation pharmaceutique suivant la revendication 1, **caractérisée en ce qu'**elle contient comme gélifiant pseudoplastique de la cellulose microcristalline, du xanthane, un polymère d'acide acrylique, de la bentonite ou un éther de cellulose choisi entre la méthylcellulose, l'hydroxypropylcellulose, la méthylhydroxypropylcellulose, la carboxyméthylcellulose sodique ou un mélange des gélifiants mentionnés.

3. Préparation pharmaceutique suivant la revendication 2, **caractérisée en ce qu'**elle contient comme gélifiant pseudoplastique de la cellulose microcristalline, de la carboxyméthylcellulose sodique, du xanthane, un polymère d'acide acrylique ou de la bentonite.

4. Préparation pharmaceutique suivant la revendication 2, **caractérisée en ce que** le gélifiant pseudoplastique est le xanthane.

5. Utilisation de préparations pharmaceutiques selon l'une des revendications précédentes pour la préparation de médicaments destinés à l'administration par voie orale.
